# EUROPEAN PATENT APPLICATION

(11) **EP 2 366 710 A2**
(43) Date of publication of application: **21.09.2011**
(21) Application number: 10013080.6
(22) Date of filing: 31.03.2006
(51) Int. Cl.: C07K 14/09, C12N 15/11

(54) **Creating poultry and other animals resistant to viral disease**

(30) Priority: 31.03.2005 US 666636 P
(62) Divisional of application: 06808899.6
(71) Applicant: Kahana, Ronen, 46497 Herzeliya (IL)
(72) Inventor: Kahana, Ronen, 46497 Herzeliya (IL)
(74) Representative: Ford, Hazel

(57) **Abstract**

The invention is directed to genetically modified animals which are resistant to viral infections. Also provided are methods for creating animals which are resistant to viral infections.

## Description

### CROSS-REFERENCE TO RELATED APPLICATIONS

This reference claims priority to U.S. Provisional Application No. 60/666,636, filed on March 31, 2005, which is herein incorporated by reference for all purposes.

### FIELD OF THE INVENTION

The invention relates to the use of RNA interference technology to create animals that are resistant to viral infections.

### BACKGROUND OF THE INVENTION

The Centers for Disease Control (CDC) and the World Health Organization have reported that the highly lethal avian influenza virus strain H5N1 has been reported in over 47 countries spread across at least 3 continents. See, for example, <http://www.cdc.gov/flu/avian/outbreaks/current.htm>. Avian influenza is an infection caused by influenza (flu) viruses. These influenza viruses occur naturally among birds. Wild birds worldwide carry the viruses in their intestines and are usually asymptomatic. However, avian influenza is very contagious among birds and can make some domesticated birds, including chickens, ducks, and turkeys, very ill and causing death.

Infected birds shed influenza virus in their saliva, nasal secretions, and feces. Susceptible birds become infected when they have contact with contaminated secretions or excretions or with surfaces that are contaminated with secretions or excretions from infected birds. Domesticated birds may become infected with avian influenza virus through direct contact with infected waterfowl or other infected poultry, or through contact with surfaces (such as dirt or cages) or materials (such as water or feed) that have been contaminated with the virus.

Infection with avian influenza viruses in domestic poultry causes two main forms of disease that are distinguished by low and high extremes of virulence. The "low pathogenic" form may go undetected and usually causes only mild symptoms (such as ruffled feathers and a drop in egg production). However, the highly pathogenic form spreads more rapidly through flocks of poultry. This form may cause disease that affects multiple internal organs and has a mortality rate that can reach 90-100% often within 48 hours. Thus, the potential for huge economic loss for poultry farms could be dramatically reduced if there were methods of creating poultry resistant to the avian flu. These virus-resistant poultry could be bred to create a new species of poultry that would pass on its viral resistance from generation to generation.

The poultry global market is estimated to produce more than 50 billions of poultry head per year. Thus, even more significant, the ability to create virus-resistant animals, such as poultry, could prevent the spread of the dreaded "bird flu" virus to humans, thus avoiding a pandemic of avian flu in humans.

Other types of viral diseases exist that are also problematic for the poultry industry. Marek's disease, for example, often causes severe death loss in pullet flocks and has been a major cause of condemnations at broiler processing plants. Marek's disease is a lymphoproliferative disease caused by MD virus (MDV) belongs to the herpesvirus family, and has been a major problem for the poultry industry during the last 50 years. Three serotypes of MDV strains are recognized. All oncogenic strains are classified as serotype 1 (MDV-1) while the naturally non-oncogenic chicken strains and herpesvirus of turkeys (HVT) belong to serotypes 2 (MDV-2) and 3, respectively. The annual economic losses due to Marek's disease throughout the world is estimated to be in the range of billion of US Dollars annually in spite of large vaccination campaigns. Although vaccination campaigns have reduced the number of affected flocks throughout the world there are large number of disease outbreaks in most parts of the world, which calls for better methods for controlling the disease.

There are limited numbers of viral diseases that can be successfully treated. Currently, vaccination is the only method used to combat viral diseases in animals. In many cases, particularly in livestock viral diseases, this approach is not applicable due to the inability to produce high efficacy vaccines. Another reason for the ineffectiveness of some vaccines is the high mutation rate, resulting in the antigenic drift or loses of vaccine effectiveness. Once a vaccine has lost its effectiveness, the disease can emerge and be able to inflict large economic losses in the case of poultry and livestock viral diseases.

There are two classical form of vaccine; one is attenuated live vaccine, which is the most potent one because it induces both-the humoral and cellular arms of the immune system. The disadvantage of this kind of vaccine is the risk of reappearing of highly virulent escapee mutants. For that reason, many countries are reluctant to approve the use of this kind of vaccine. The second type is inactivated viral vaccine, which is in many cases much less effective since it only induces the humoral immune system. In the last few years, several types of recombinant vaccines such as subunit vaccines and DNA vaccine have been developed. Unfortunately, most of them are not in wide spread use.
One of the disadvantages of vaccination is the high annual economical expenditure as demonstrated in the case of Marek's Disease. According to the World Organisation for Animal Health (OIE), the total number of animals that have been vaccinated against Marek's disease in 2002 was 2.457 billion. See, for example, <www.oie.com>.

There are two known ways to vaccinate. The first method is a cell-free (lyophilized) form costing about $3/1000 units. The second form is a cell-associated ("wet") form costing about $8/1000 unit. The estimated world expenditure on vaccination for Marek's disease in 2002 was approximately between $7.4 billion to $19.7 billion.

Since the early 1970s most chickens have been vaccinated against MD using attenuated serotype 1 strains or HVT. Starting in 1983 bivalent and polyvalent combinations has been used to protect against the more virulent field strains. Although vaccination is remarkably effective in protecting chickens, MD remains high on the list of disease priorities. The main reason for the high priority is the continuous evolution of MDV strains towards increased virulence causing vaccine breaks.

Accordingly, there is a need for a method of preventing or lessening the transmittal of viral diseases in animals, for example, in avian influenza and Marek's disease in poultry. Given the current outbreak of avian influenza virus, a strong need exists for methods that can create genetically modified animals, such a poultry, that are resistant to viral infections, such as avian influenza.

In the past decade, a new approach for gene inactivation via gene silencing, termed "RNA interference" (RNAi) has been disclosed. See, for example, Fire et al., Nature 391: 806-811 (1998) and U.S. Patent 6,506,559. RNA interference refers to an event which occurs when an RNA polynucleotide acts through endogenous cellular processes to specifically suppress the expression of a gene whose sequence corresponds to that of the RNA. The silencing of the target gene occurs upon the degradation of mRNA by double strand (ds) RNA by the host animal, sometimes through RNAase III Endonuclease digestion. The digestion results in molecules that are about 21 to 23 nucleotides (or bases) in length (or size) although molecular size may be as large as 30 bases.

These short RNA species mediate the degradation of corresponding RNA messages and transcripts, possibly via an RNAi nuclease complex, called the RNA-induced silencing complex (RTSC), which helps the small dsRNAs recognize complementary mRNAs through base-pairing interactions. Following the siRNA interaction with its substrate, the mRNA is targeted for degradation, perhaps by enzymes that are present in the RISC. This type of mechanism appears to be useful to the organisms in inhibiting viral infections, transposon jumping, and similar phenomena, and to regulate the expression of endogenous genes. RNAi activity have been so far documented in plants, insects, nematodes and vertebrates among other organisms. For general background information, see, for example, Schutz et al., Virology 344(1):151-7 (2006); Leonard et al., Gene Ther. 13(6):532-40 (2006); Colbere-Garapin et al., Microbes Infect. 7(4):767-75 (2005); Wall, Theriogenology 57(1):189-201 (2002); El-Bashir, et al., Nature 411: 494-498 (2001); Fire, A., et al. Science 391: 806-811 (1998); Gitlin et al., Nature 418: 430-434 (2002); Gitlin, et al., J. Virol. 79:1027-1035 (2005); Kahana, et al., J. Gen. Virol. 85, 3213-3217 (2004); Kronke et al., J. Virol. 78: 3436-3446 (2004); Leonard et al., J. Virol. 79:1645-1654 (2005); and Yokota, et al., EMBO Rep. 4: 602-608 (2003).

Given the widespread problem of various viral diseases in animals, poultry in particular, a different approach to effectively control poultry and livestock viral diseases would be very beneficial to solving this problem. As such, the invention disclosed herein provides a solution to this problem by providing for methods to create transgenic non-human vertebrates, for example, poultry and livestock, carrying and expressing molecules targeted to block important viral functions that will significantly inhibit virus replication of pathogenic viruses as well as the genetically modified poultry and livestock itself.

### BRIEF SUMMARY OF THE INVENTION

The invention provides for germ cells encoding for siRNA that target viral sequences and methods of obtaining the same. The invention also provides for non-human vertebrates that are resistant to viral infection and methods of obtaining the same.

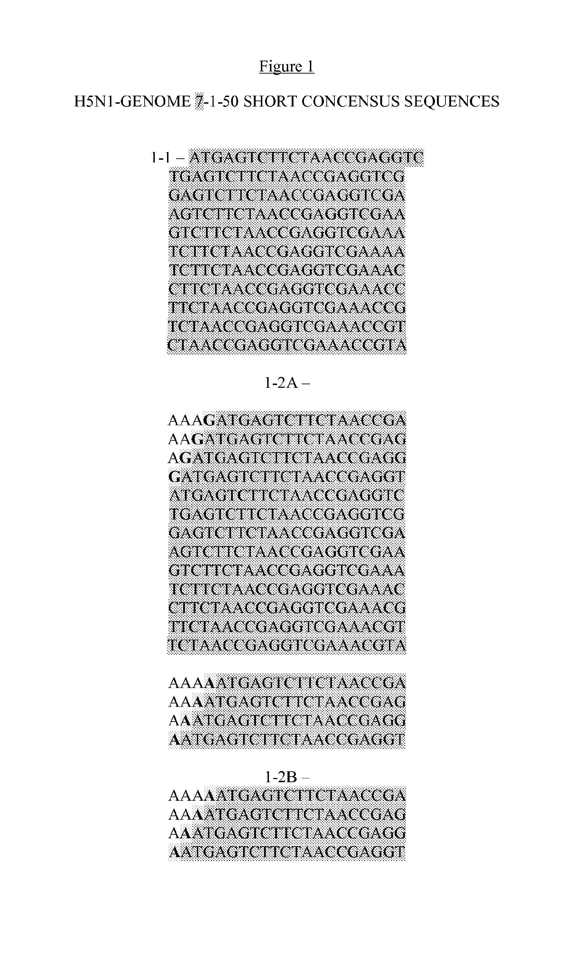

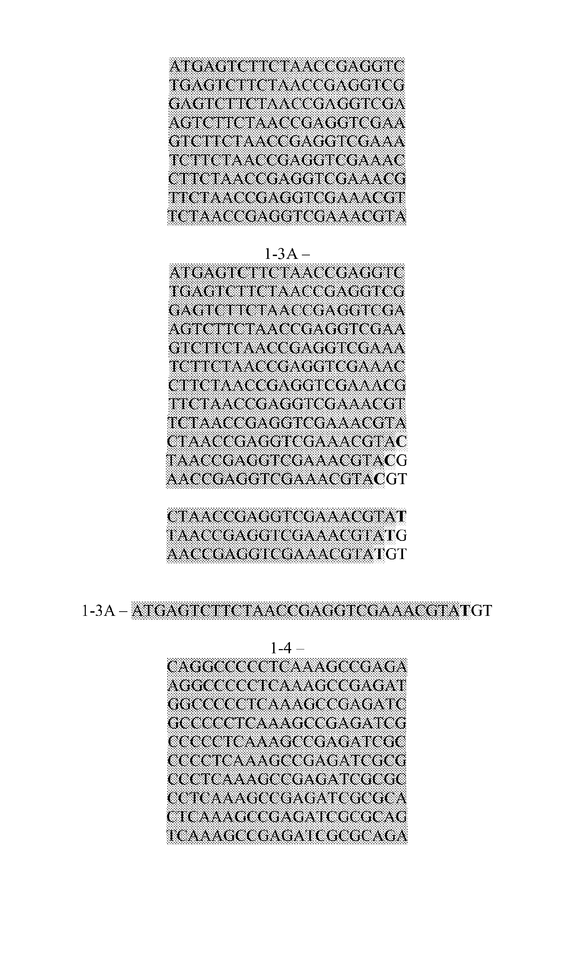

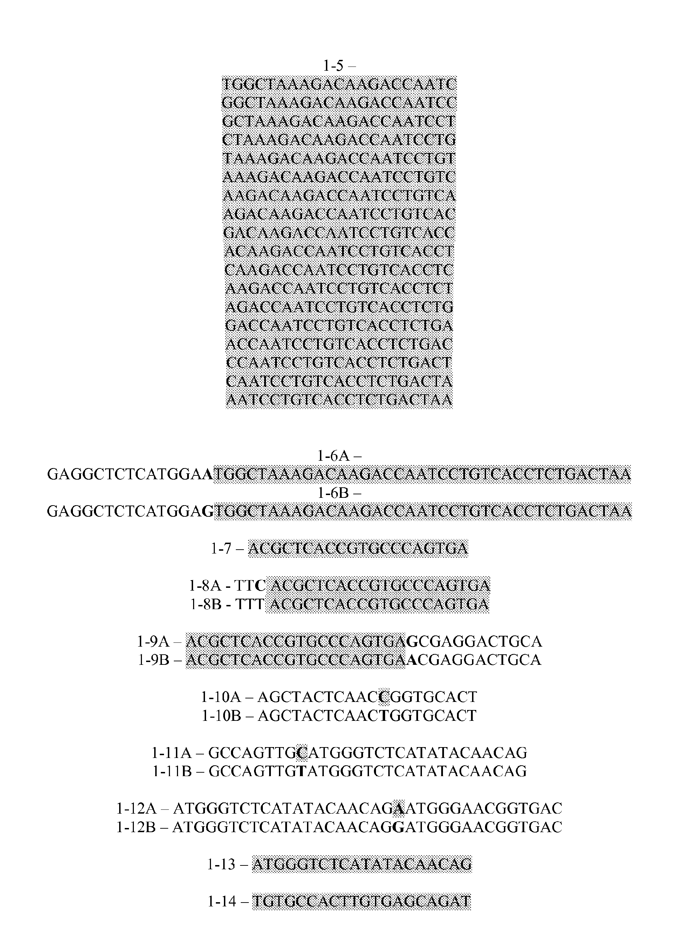

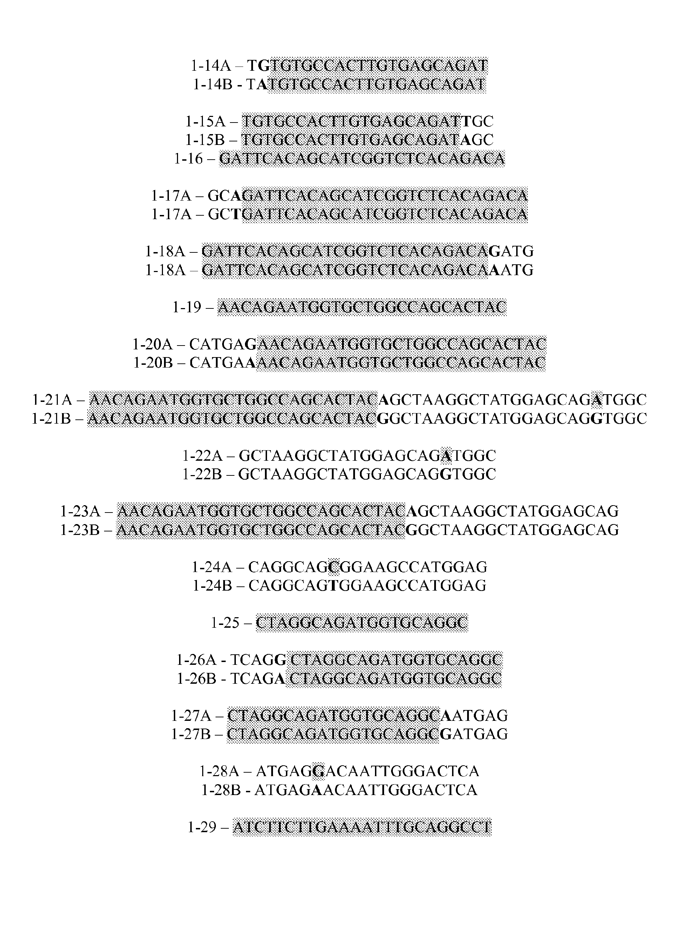

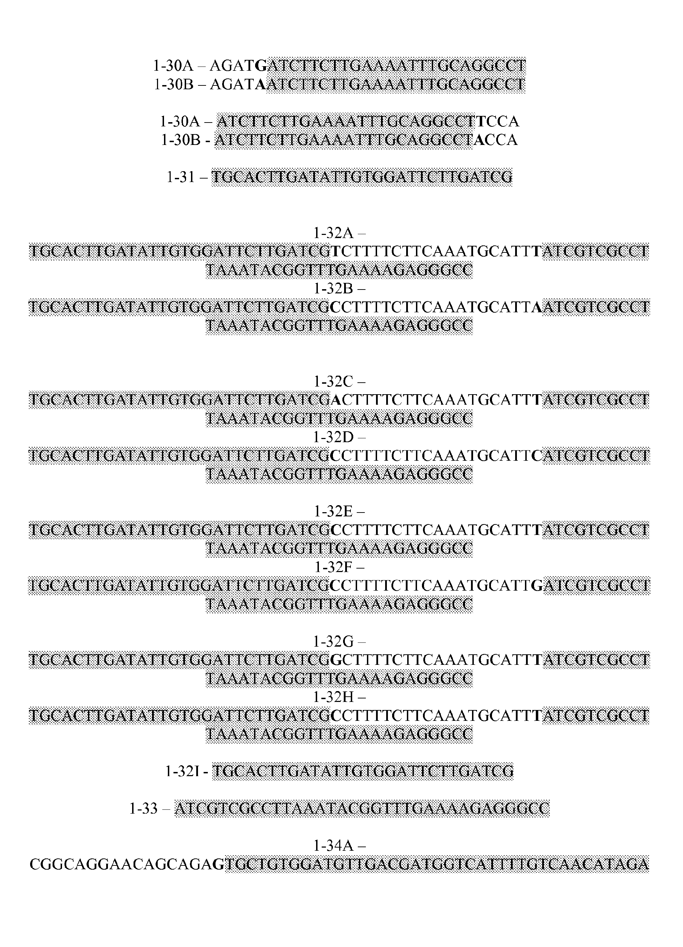

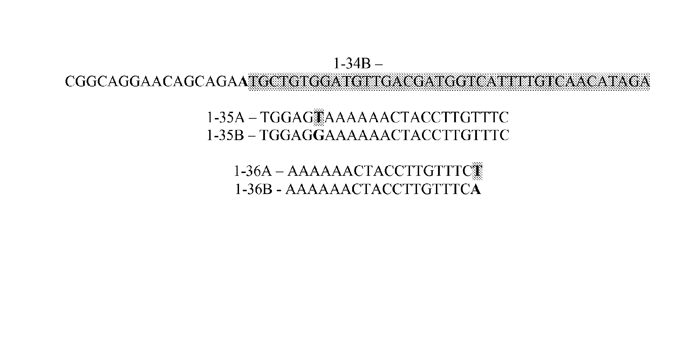

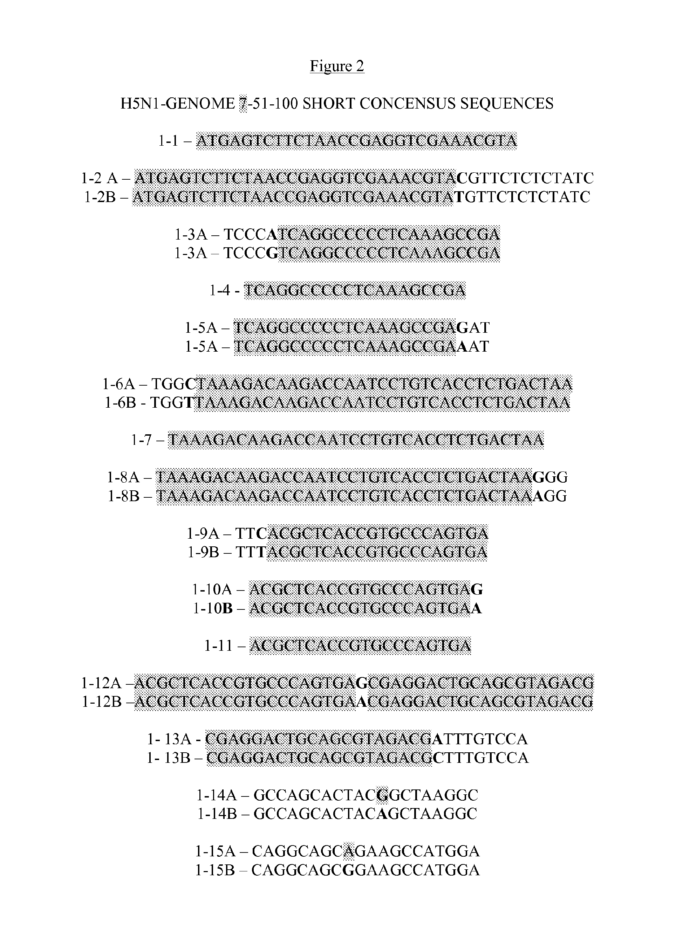

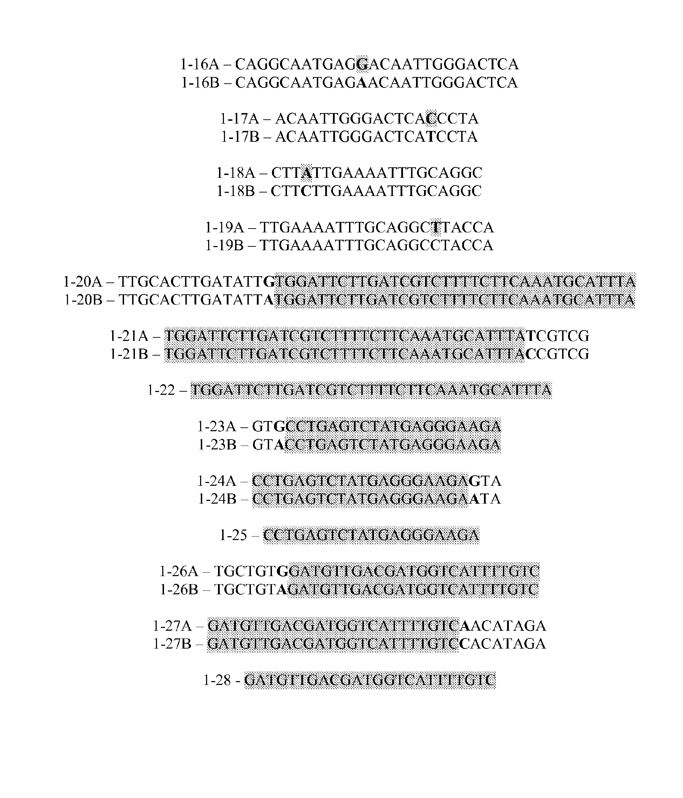

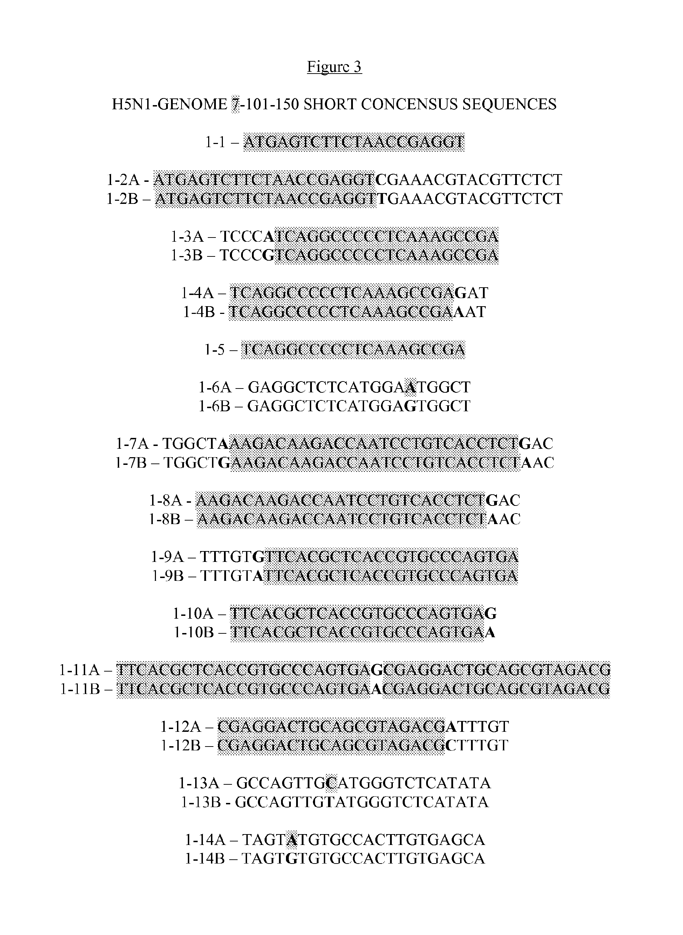

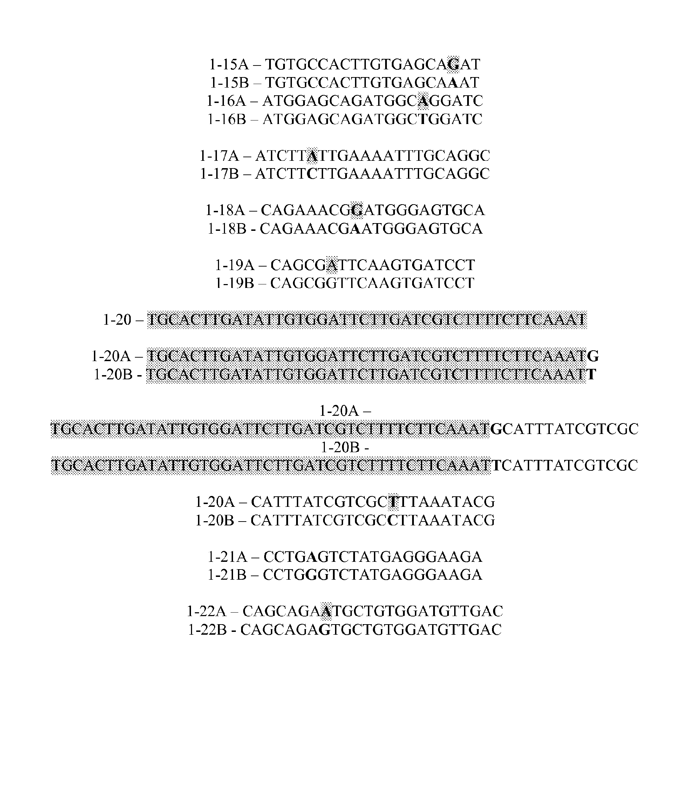

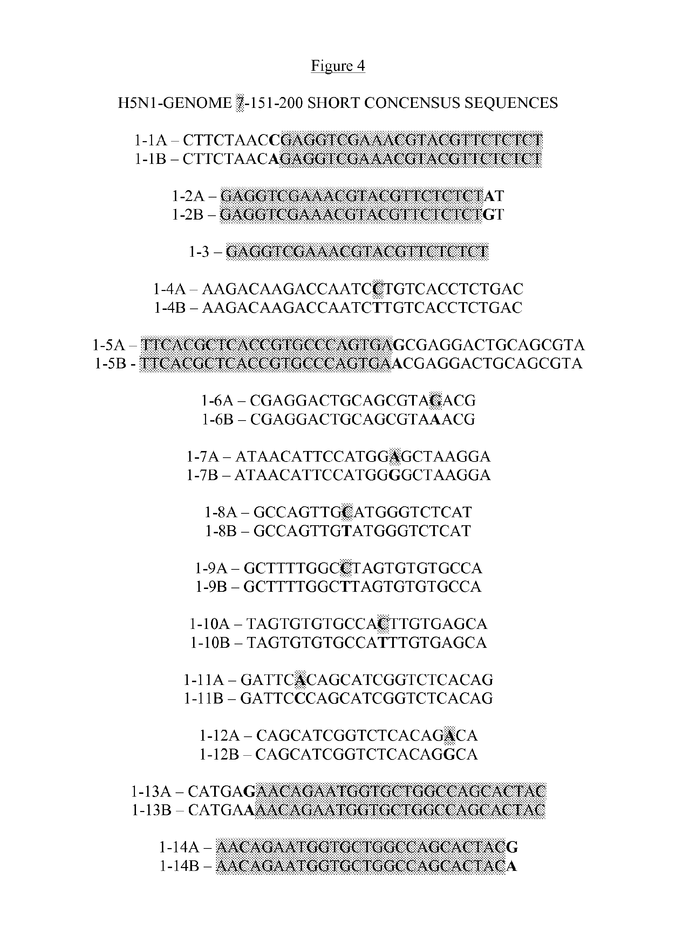

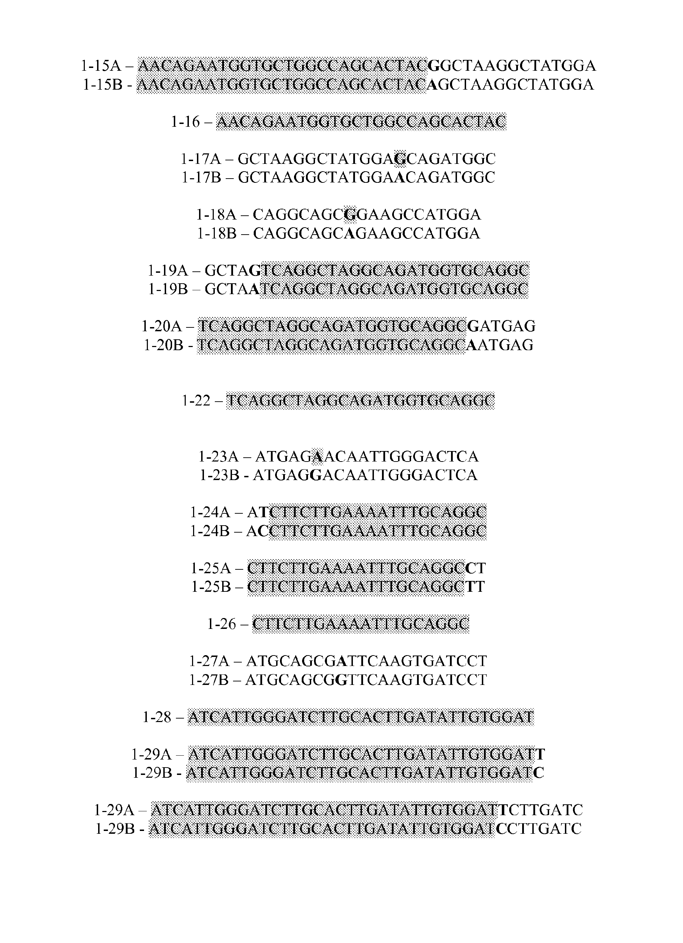

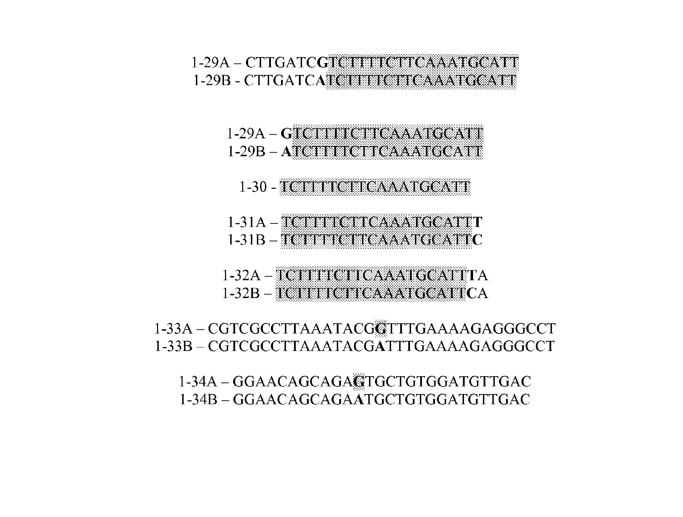

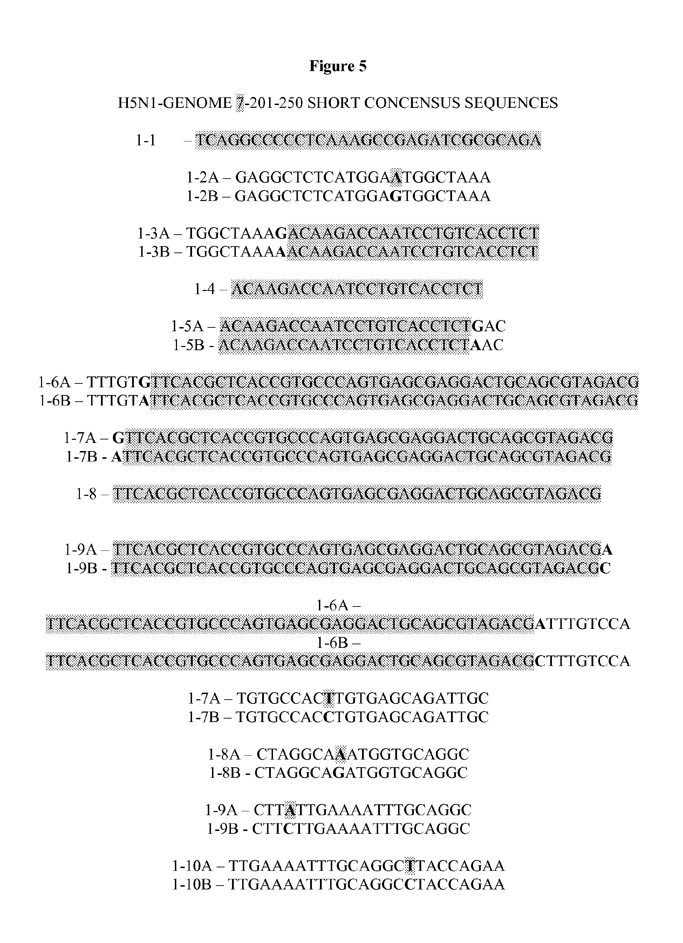

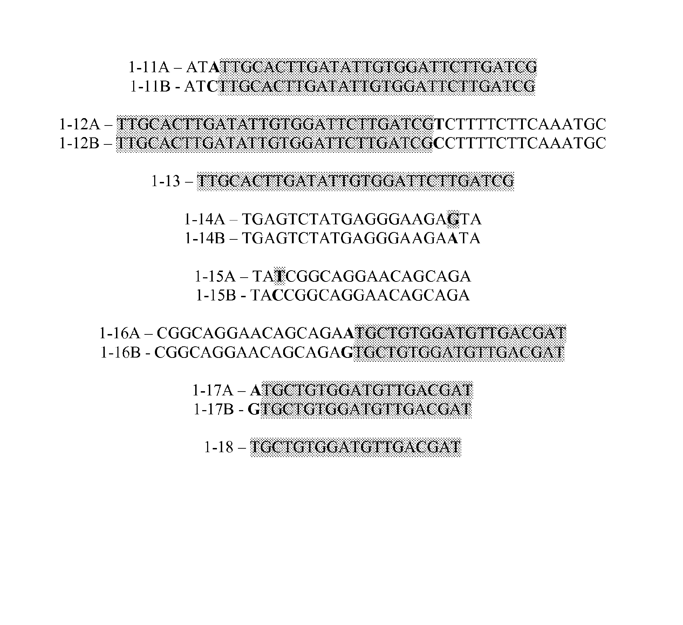

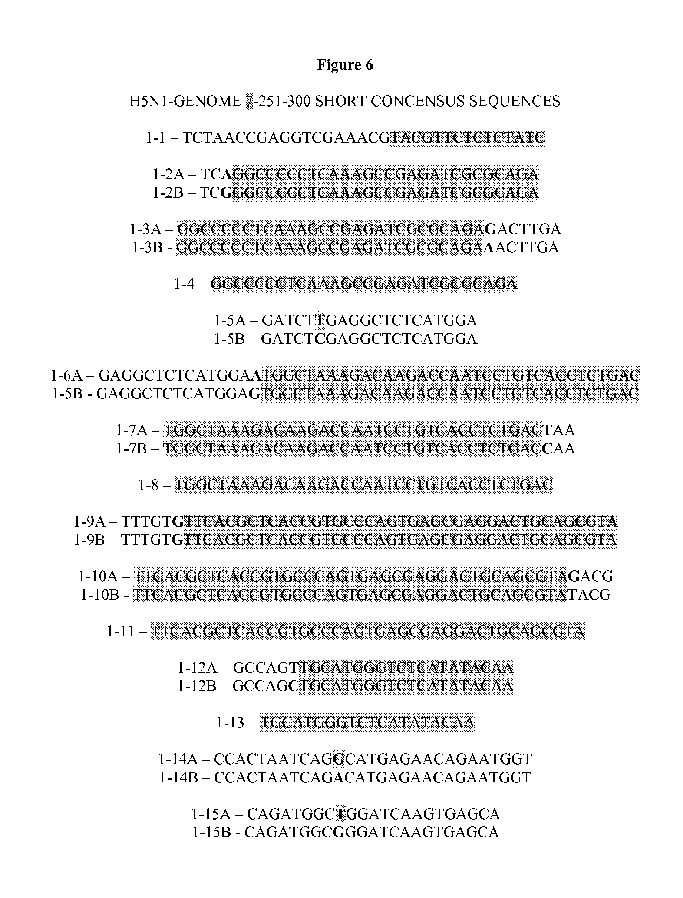

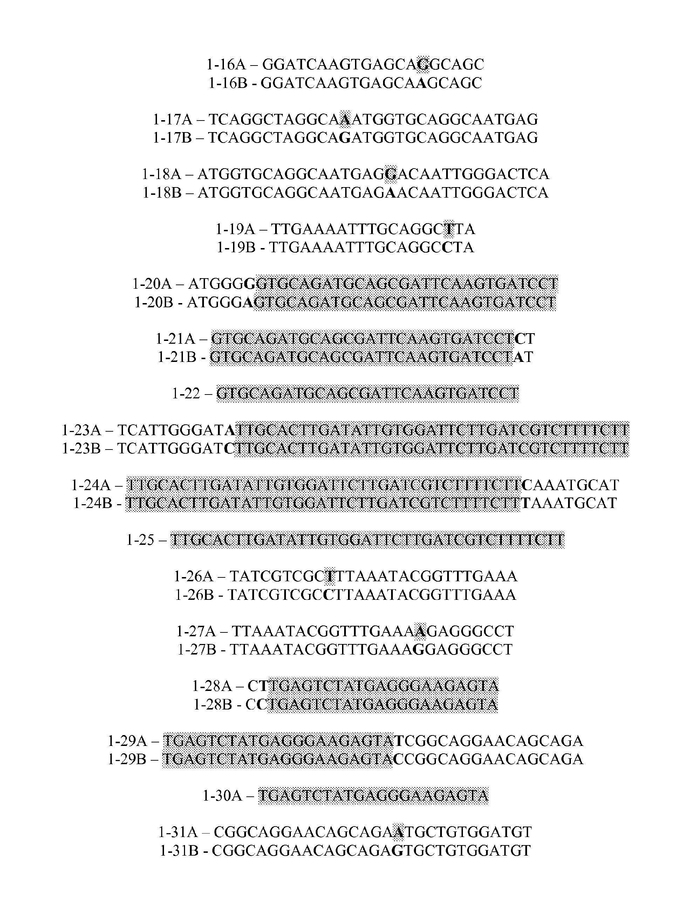

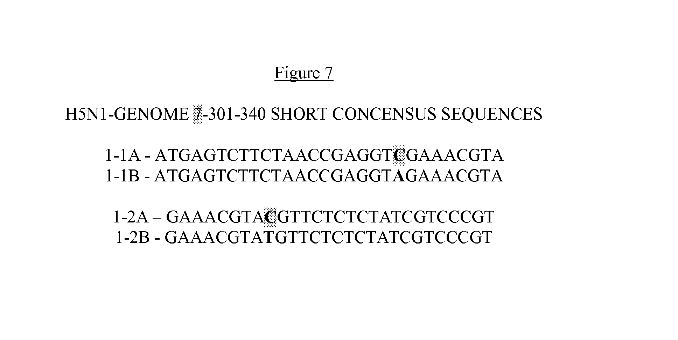

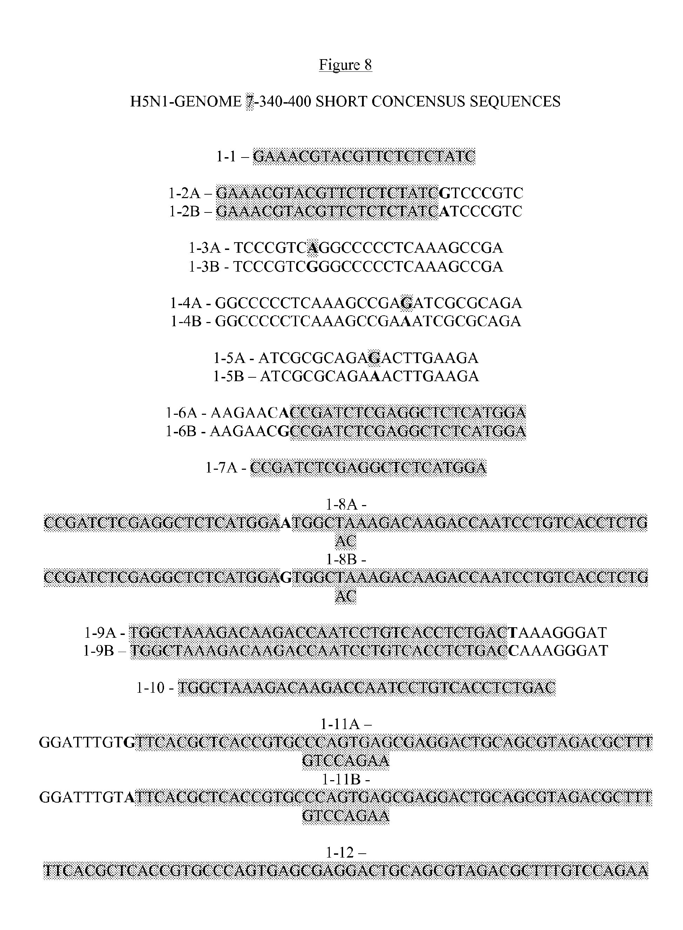

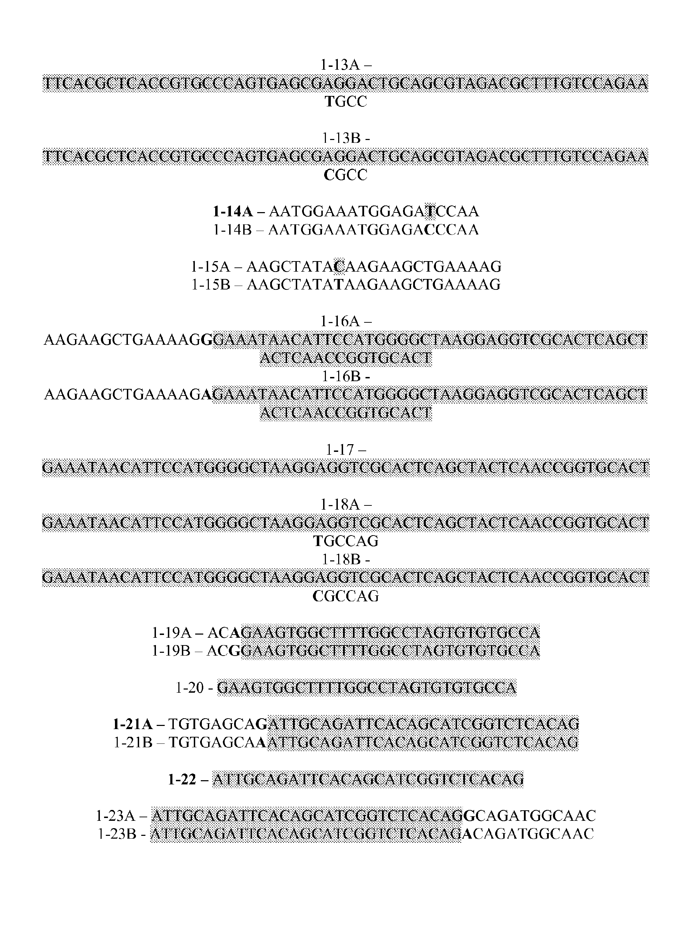

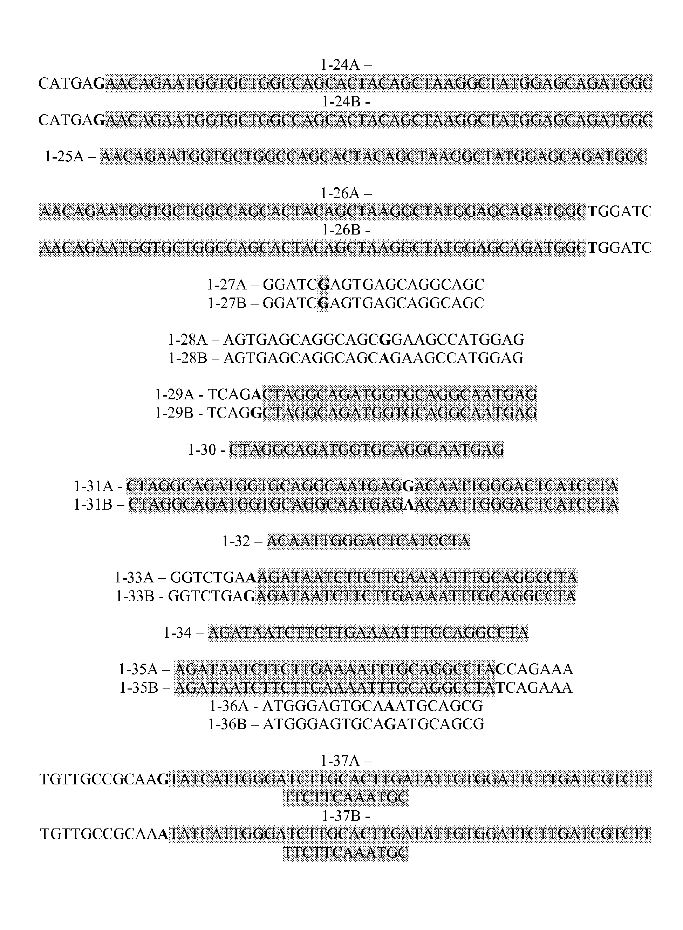

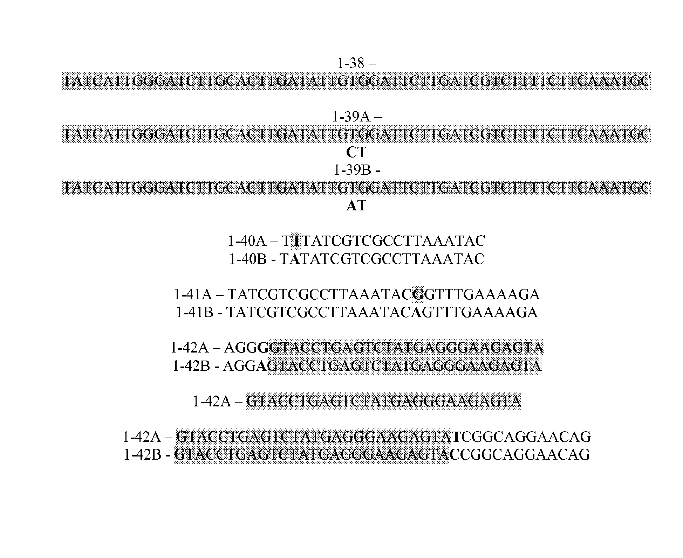

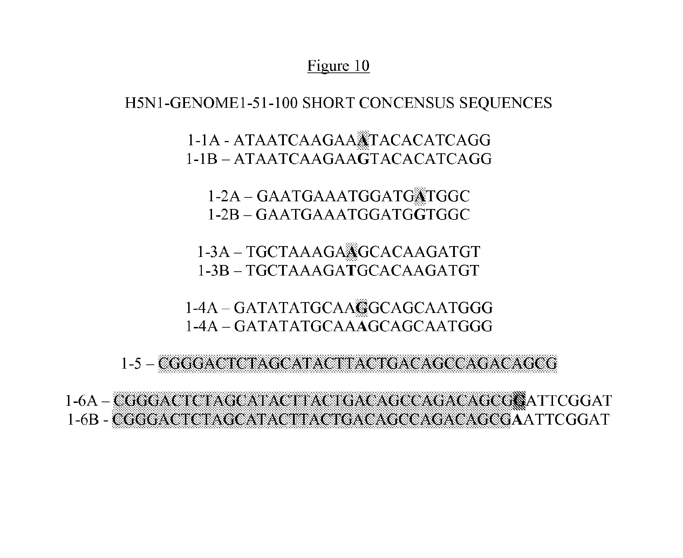

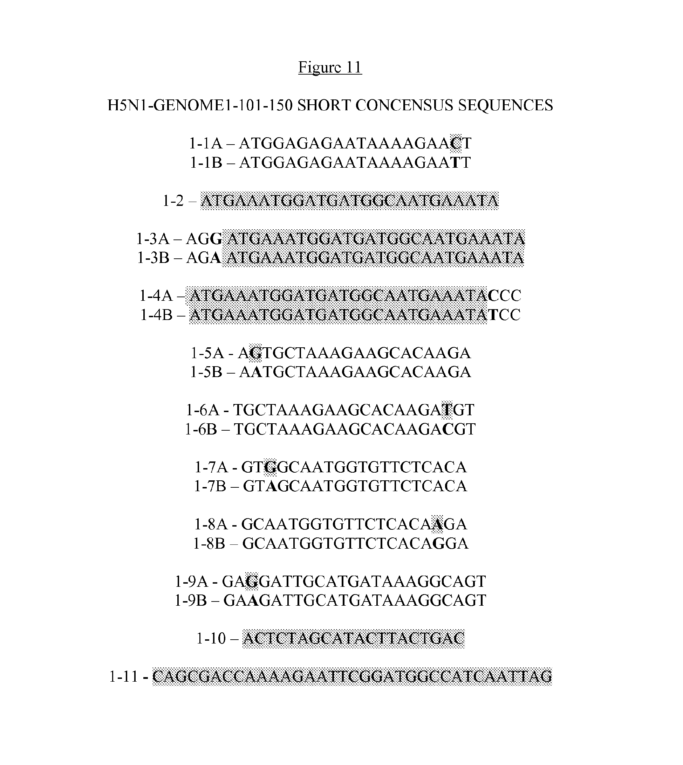

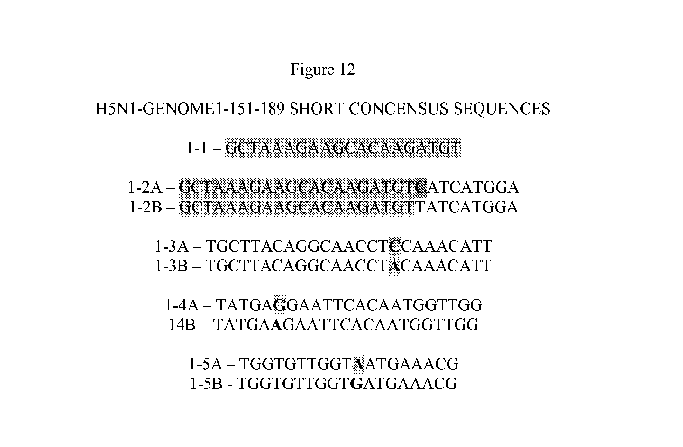

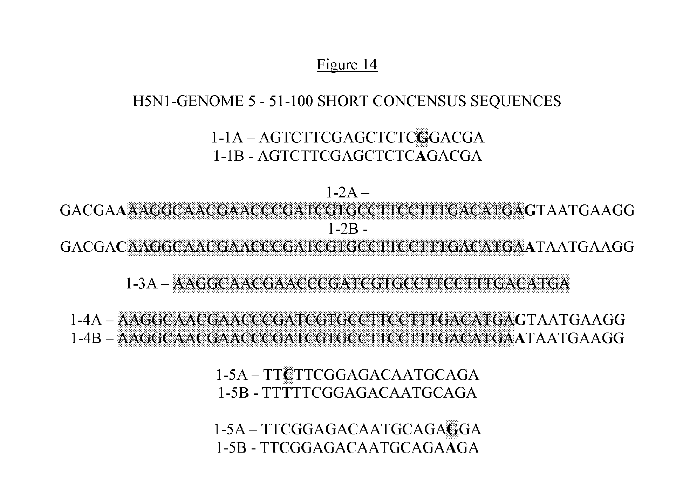

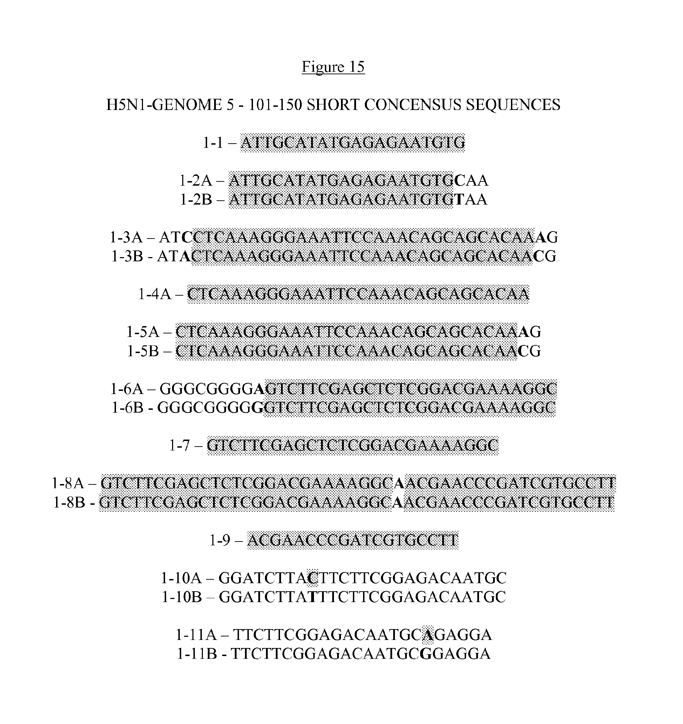

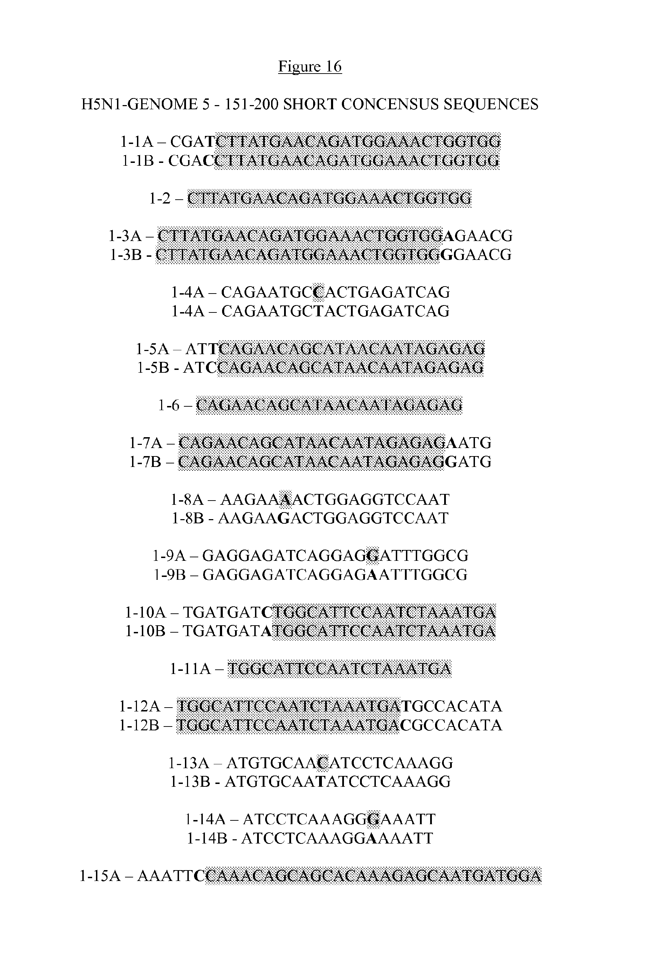

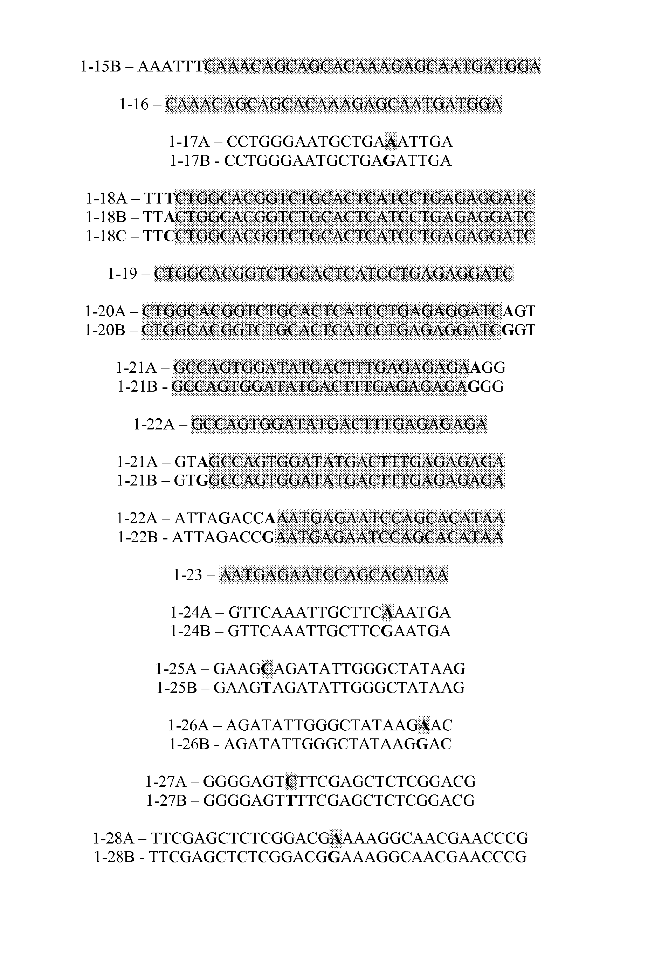

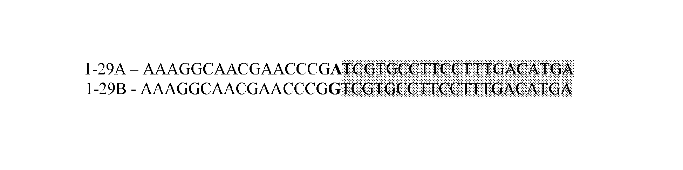

In one aspect, the invention is a germ cell of a non-human vertebrate containing a construct comprising a sequence encoding an siRNA to a conserved region of a viral genome, wherein the sequence is operably linked to a promoter. In one embodiment, the construct comprises sequences encoding multiple siRNAs to the viral genome. In another embodiment, the cell contains multiple constructs comprising a sequence encoding multiple siRNAs to conserved regions of the viral genome, wherein the sequence is operably linked to a promoter. In another embodiment, the vertebrate is a non-human mammal. In another embodiment, the virus is foot-and-mouth disease virus (FMDV). In another embodiment, the conserved sequence is selected from the group consisting of SEQ ID NO: 1, SEQ ID NO: 2, and SEQ ID NO: 3. In another embodiment, the vertebrate is of an avian species. In another embodiment, the virus is avian influenza virus. In another embodiment, the conserved sequence is selected from the group consisting of the sequences in Figures 1-16. In another embodiment, the virus is Marek's disease virus (MDV). In another embodiment, the germ cell is sperm.

In another aspect, the invention provides for a non-human vertebrate that is resistant to a viral disease, wherein the majority of cells in the vertebrate comprise a sequence encoding an siRNA to a conserved region of a genome of a virus causative of the disease, wherein the sequence is operably linked to a promoter. In one embodiment, the construct comprises sequences encoding multiple siRNAs to conserved regions of the viral genome. In another embodiment, the cell contains multiple constructs comprising a sequence encoding multiple siRNAs to conserved regions of the viral genome. In another embodiment, the vertebrate is a non-human mammal. In another embodiment, the viral disease is FMDV. In another embodiment, the conserved sequence is selected from the group consisting of SEQ ID NO: 1, SEQ ID NO: 2, and SEQ ID NO: 3. In another embodiment, the vertebrate is of an avian species. In another embodiment, the virus is avian influenza virus.. In another embodiment, the conserved sequence is selected from the group consisting of the sequences in Figures 1-16. In another embodiment, the virus is FMDV.

In another aspect, the invention provides for a method of generating a germ cell of a non-human vertebrate comprising incubating the germ cell with a construct comprising a sequence encoding an siRNA to a conserved region or a viral genome wherein the sequence is operably linked to a promoter under conditions that cause the construct to be taken into the cell. In one embodiment, the construct is integrated into the host cell genome.

In another aspect, the invention provides for a method for generating a non-human vertebrate that is resistant to viral disease comprising (a) incubating the germ cell with a construct comprising a sequence encoding an siRNA to a conserved region or a viral genome wherein the sequence is operably linked to a promoter under conditions that it forms a diploid cell; and (b) incubating the diploid cell of (a) under conditions that it forms a non-human vertebrate.

### BRIEF DESCRIPTION OF THE DRAWINGS

Figures 1-8 show the avian influenza H5N1 sequences that can be used for genome 7 of this avian influenza strain.

Figures 9-12 show the avian influenza H5N1 sequences that can be used for genome 1 of this avian influenza strain.

Figures 13-16 show the avian influenza H5N1 sequences that can be used for genome 5 of this avian influenza strain.

### DETAILED DESCRIPTION OF THE INVENTION

The present invention provides for animals which are resistant to viral infections and methods of creating these types of transgenic animals. The target viral genes will be silenced by the presence of silencing molecules, such as siRNA molecules disclosed herein, and thus, will be unable to perform their role in viral life cycle. The outcome of this process will be a significant block of virus replication leading to the inability of the infection to induce morbidity or/and mortality in these animals. Thus, this invention will have a substantial economic effect for the farmers as well as for society as a whole ensuring constant supply of meat and other livestock products.

The practice of the present invention will employ, unless otherwise indicated, conventional techniques of molecular biology (including recombinant techniques), microbiology, cell biology, biochemistry and immunology, which are within the skill of the art. Such techniques are explained fully in the literature, such as, Molecular Cloning: A Laboratory Manual, second edition (Sambrook et al., 1989) Cold Spring Harbor Press; Oligonucleotide Synthesis (M.J. Gait, ed, 1984); Animal Cell Culture (R.I. Freshney), ed., 1987); Methods in Enzymology (Academic Press, Inc.); Handbook of Experimental Immunology (D.M. Weir & C.C. Blackwell, eds.); Gene Transfer Vectors for Mammalian Cells (J.M. Miller & M.P. Calos, eds., 1987); Current Protocols in Molecular Biology (F.M. Ausubel et al., eds., 1987); PCR: The Polymerase Chain Reaction, (Mullis et al., eds., 1994); Current Protocols in Immunology (J.E. Coligan et al., eds., 1991) and Short Protocols in Molecular Biology (Wiley and Sons, 1999).

All references, patent, and patent applications cited in this patent application are herein incorporated by reference, each in its respective entirety for all purposes.

### Definitions

All scientific and technical terms used in this application have meanings commonly used in the art unless otherwise specified. As used.in this application, the following words or phrases have the meanings specified.

The terms "polynucleotide" and "nucleic acid", used interchangeably herein, refer to a polymeric form of nucleotides of any length, either ribonucleotides or deoxyribonucleotides. These terms include a single-, double- or-triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases.

A "promoter" is a control sequence that is a region of a polynucleotide sequence at which initiation and rate of transcription are controlled. It may contain genetic elements at which regulatory proteins and molecules may bind such as RNA polymerase and other transcription factors. Promoters may be constitutive, inducible, repressible, or tissue-specific, for example. The phrases "operable linked," "operatively positioned," "operatively linked," "under control," and "under transcriptional control" mean that a promoter is in a correct functional location and/or orientation in relation to a nucleic acid sequence to control transcriptional initiation and/or expression of that sequence. A promoter may or may not be used in conjunction with an "enhancer," which refers to a cis-acting regulatory sequence involved in the transcriptional activation of a nucleic acid sequence. One promoter may regulate the expression of one or more genes.

### RNAi Targets

The invention provides for methods for silencing genes such that virally-resistant animals can be produced. In one embodiment, the invention uses genes silencing technology, such as RNAi, associated with lipofection to create internally immune transgenic poultry and livestock. In a preferred embodiment, the invention uses Restriction Enzyme Mediated Integration ("REMI"), as disclosed, for example, in WO 99/42569, to create virally resistant animals. The animals will carry and express a single or multiple genes silencing molecules targeted to viral genes (e.g., replication indispensable viral genes).

The short interfering RNA ("siRNA") provided by the present invention allows for the modulation and/or the attenuation of target gene expression when such a gene is present and capable of expression within a cell. Modulation of expression can be partial, more preferably a complete inhibition, of gene function, or even the up-regulation of other, secondary target genes or the enhancement of expression of such genes in response to the inhibition of the primary target gene.

Attenuation of gene expression may include the partial or complete suppression or inhibition of gene function, transcript processing or translation of the transcript. In the context of RNA interference, modulation of gene expression is thought to proceed through a complex of proteins and RNA, specifically including small, dsRNA that may act as a "guide" RNA. The siRNA therefore is thought to be effective when its nucleotide sequence sufficiently corresponds to at least part of the nucleotide sequence of the target gene. Although the present invention is not limited by this mechanistic hypothesis, it is highly preferred that the sequence of nucleotides in the siRNA be substantially identical.to at least a portion of the target gene sequence. In one embodiment the sequence identity between the target viral nucleotide and the siRNA is 100%, *i.e.,* exact homology. In another embodiment, there is a 1 base pair (bp) difference. In another embodiment, there is a 2 base pair difference. In another embodiment, there is a 3 base pair difference.

A "target gene" or "target sequence" generally means a polynucleotide comprising a region that encodes a gene product, such as a polypeptide, or a polynucleotide region that regulates replication, transcription or translation or other processes important to the expression of the polypeptide, or a polynucleotide comprising both a region that encodes a polypeptide and a region operably linked thereto that regulates expression. The target sequence does not necessarily have to code for the entire gene product. In the context of the invention, "operably linked" refers to the promoter being in a correct functional location and/or orientation in relation to a polynucleotide sequence to control initiation and/or expression of that sequence.

In one aspect, the target genes for the siRNA are viral genes. In one embodiment, the viral genes are those involves in the propagation of avian viral diseases (e.g., avian influenza and Marek's disease). Gene sequences which are encompassed within the scope of this invention include those shown in Figures 1-16.

The siRNA is of a length sufficient to significantly block virus replication but not to cause cell destruction and are usually in the range of between about 19 base pairs to about 27base pairs. In one embodiment, the siRNA is 21 bp is length. In another embodiment, the siRNA is 22 bp in length. In yet other embodiments, the siRNA are about 19, 20, 23, 24, 25, 26, or 27 bp in length. In yet another embodiment, the siRNA are about 28, 29, or 30 bp in length. It will be apparent to one of skill in the art that the length of the siRNA cannot be too long because it will trigger a type of self-destruction mechanism for the cell in which the siRNA resides. For example, Elbashir et al. teaches that a length of 21 bp in length to be effective in evading the anti-viral mechanisms of the cell, such as interferon response (see Elbashir et al., Nature 411:494-498 (2001)).

As shown in Figures 1-16, varying lengths can be used as interfering RNA. One preferred embodiment is 21 bp in length. One of skill in the art can use the sequences depicted in Figures 1-16 and move stepwise down the sequence one base pair at a time to come up with a different 21 bp siRNA. Some of the permutations are shown in these figures. However, not all the possible 21 bp siRNA sequences are shown because once the general sequence has been shown, as done in Figures 1-16, it would routine for one of skill in the art to move stepwise down the sequence. In addition, lengths other than 21 bp are also encompassed within this invention. As discussed above; in one embodiment, the siRNA are about 21 bp to about 27 bp in length. A skilled artisan would use the sequences disclosed in Figures 1-16 and use the length desired and move down the sequence one bp at a time to generate a different frame. For example, if a 23 bp siRNA were to be made, then a skilled artisan would start at one end of the sequence and use the first 23 bp as one permutation of an siRNA. Then, he/she would move one base pair down the sequence and the next 23 base pair would be the next permutation and so forth.

Sequence data, either experimentally determined or accessible from public databanks can be screened by bioinformatic tools. Homology analysis is accomplished by using publicly available or commercially available programs (e.g., PILEUP and PRETTY programs in the GCG package). Homology searches are optionally conducted with publicly available sequences (e.g., Genbank) using the PILEUP and PRETTY programs or FASTA program (Pearson et al., PNAS 85: 2444-2448 (1988)).

Viral gene sequences, especially those that affect the health and safety of animals, are contemplated as target genes in this invention. The ideal viral sequences that serve as targets are those that are highly conserved. The high degree of conservation generally is a indication of selective pressure not to mutate away from the conserved sequence. For many viruses, non-structural genes tend to be highly conserved across its various serotypes and therefore, are good targets for RNAi technology. Other viral genes that are also good targets are those genes that are important or essential for viral replication and subsequent propagation. In one aspect, the structural (S) gene of the Akabane virus is used to create siRNA.

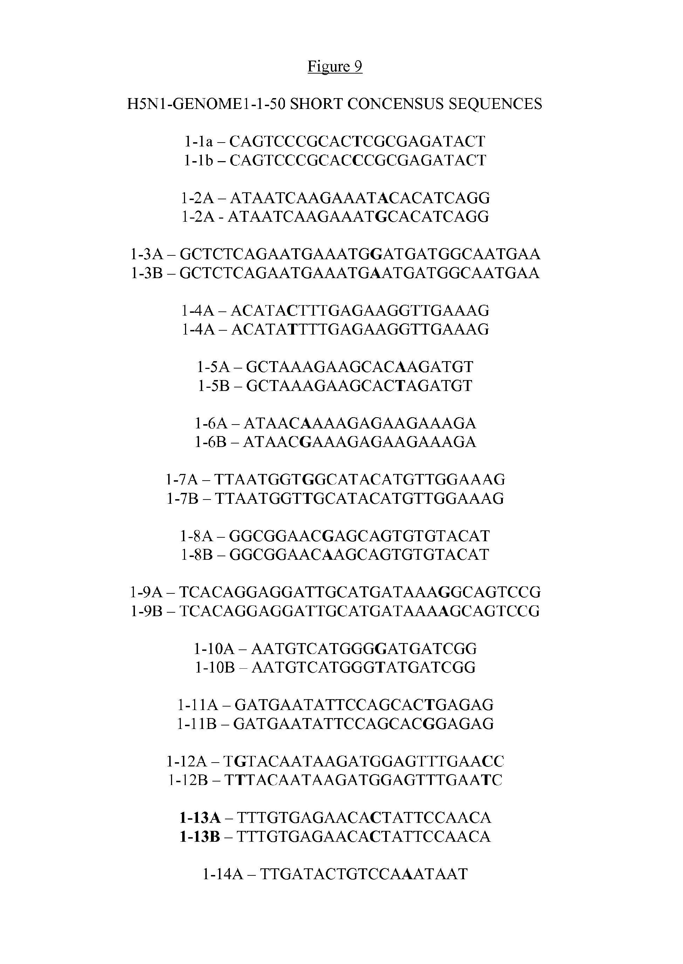

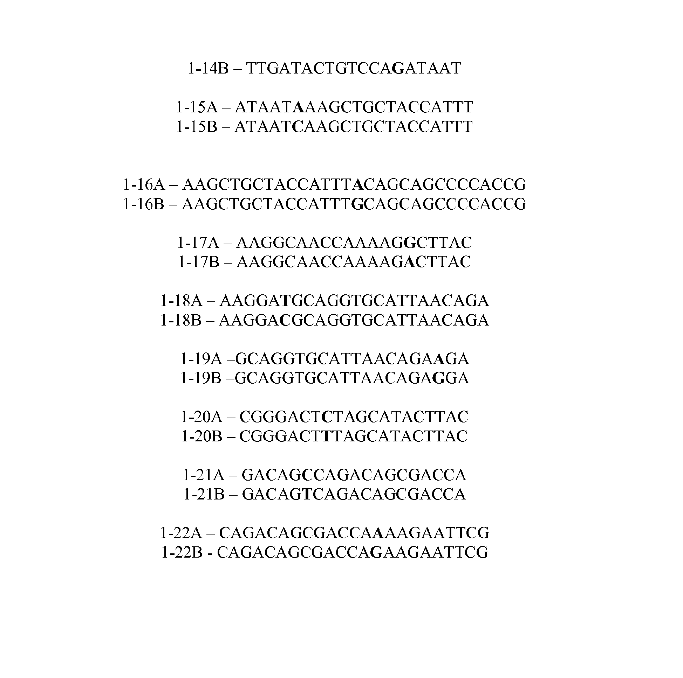

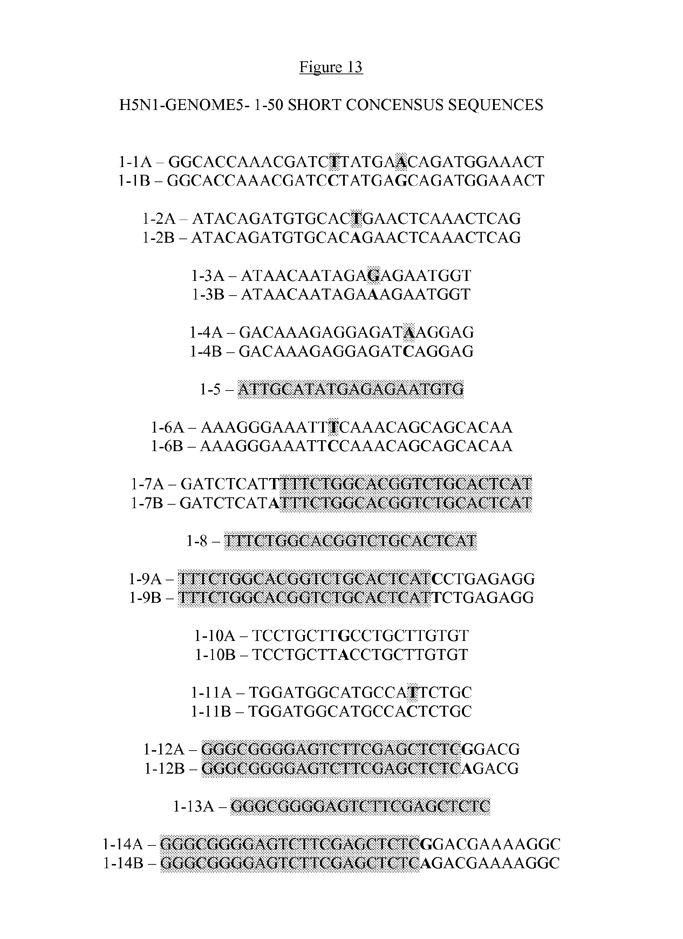

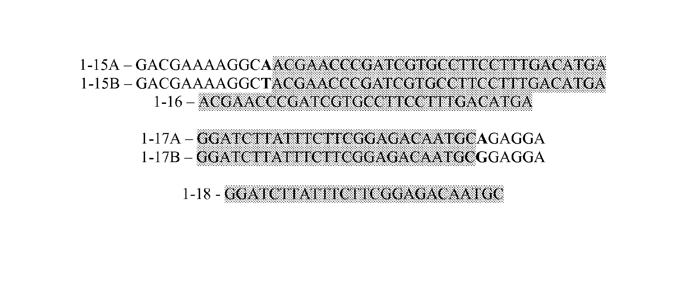

In other aspects, target sequences are short stretches of sequence that are conserved across different strains of avian influenza. In one embodiment, the sequence used for siRNA is from genome 1 of the H5N1 strain of the avian influenza virus. Non-limiting examples of this is shown in Figures 9-12. The bolded nucleotide(s) indicates where a base pair difference(s) was detected when a homology analysis was conducted. In another embodiment, the target sequence used for siRNA is from genome 5 of the H5N1 strain of the avian influenza virus. Non-limiting examples of this is shown in Figures 13-16. In another embodiment, the target sequence used for siRNA is from genome 7 of the H5N1 strain of the avian influenza virus. Non-limiting examples of this is shown in Figures 1-8. In other embodiments, the target sequence is from other genome types of H5N1. Furthermore, siRNA to combat other strains of avian influenza (e.g., H5N2, H5N3, etc.) are also encompassed within the scope of this invention and can be made by a skilled artisan by following the teachings herein.

In another aspect, conserved stretches of foot-and-mouth disease virus (FMDV) are used as target sequences for constructing the siRNA. In yet other aspects, a non-limiting list of the viruses which cause the following diseases are used as target sequences for siRNA construction: Newcastle disease, West Nile fever, fowlpox, avian infectious bronchitis, avian encephalomyelitis, avian leukosis, duck virus hepatitis, duck virus enteritis, lumpy skin disease, infectious bovine rhinotracheitis, bovine virus diarrhoea, bovine leukosis, Rift Valley fever, Rinderpest, Bluetongue, Peste des petits ruminants (PPR), sheep pox and goat pox, contagious pustular dermatitis (ecthyma), border disease, Maedi-visna, transmissible Gastro Enteritis(TGE), equine influenza, African horse sickness, Venezuelan equine encephalomyelitis, and spring viremia of carp.

### Constructs Encoding siRNA

Constructs encoding siRNA involve several components, including but not limited to promoters and sequences which will "guide" the siRNA to the correct target in the cell. Promoters which may be used include, but are not limited to, RNA polymerase II and RNA polymerase III promoters. RNA pol III promoters work especially well as promoters for the construction of siRNA. Non-limiting examples of promoters (both pol II and pol III) which may be used include: 5S ribosomal (r) RNA, mouse U6, human U6, mouse HI, human HI, cytomegalovirus promoter, chicken ubiquitin C, human 7SK promoter, bovine U6 promoter, chicken U6 promoter, Marek's disease virus 38 kd phosphorylated protein (pp38) gene, 1.8-kb mRNA chicken, human, bovine beta-actin, chicken PRL promoter, chicken SPATA4 genes promoter, chicken PolI promoter, US1 gene of Marek's disease virus promoter, Marek's disease virus small subunit of ribonucleotide reductase gene, avian leukemia and sarcoma viruses LTR, RSV-LTR, synthetic poxvirus promoters, vaccinia virus P11 promoter, vaccinia virus P 174 and P 190, fowlpox early/late promoter P.E/L, fowlpox virus thymidine kinase promoter, vaccinia p7.5 promoter, and fowlpox-PFL1 promoter.

Using standard molecular biology techniques, these promoters are operably linked to the sequences that will target viral genes and silence them. One promoter can regulate the expression of one or more target sequences. The promoter(s) and target sequence(s) are cloned into standard cloning or expression vectors. As used herein, "vector" refers to nucleic acid molecules that are capable of delivering other nucleic acid sequences to a cell. Vectors can be derived from plasmids, bacteriophages, .plants or other animal viruses.

siRNA constructs are made such that they are capable of expressing the siRNA. The skilled artisan will appreciate that certain types of promoters will regulate expression of siRNA better in some vertebrates than in other vertebrates, depending on the physiology of the vertebrate and should take steps to use the promoter that is best suited for that vertebrate. A promoter can also regulate the expression of one or more siRNA. A vector can contain sequences that encode for one or more siRNAs. Enhancers, selectable markers and other standard molecular tools can also be used for easier molecular manipulation. When using the REMI technique, the vector should be constructed such that it facilitates the use of the REMI technique. Appropriate guidance for that technique is found in WO 99/42569.

In one aspect of the invention, a vector may be used with several siRNA genes that correspond to different targets on the viral genome. This type of vector construction will ensure significant to complete inhibition due to simultaneous cleavage at various sites. Further, it will still be active even if the one of the viral target sequence mutates or changes. For the double-stranded siRNA, it is possible to have the standard 2 bp overhang, as well as a 1 bp overhang or no overhang at all.

### Construction of Germ Cells

As used herein, a "germ cell" is defined as sperm and egg cells and their precursors. Germ cells are haploid and have only one set of chromosomes while other non-germ cells have two sets of chromosomes. The present invention provides for construction of a germ cell of a non-human vertebrate containing a construct which comprises a sequence encoding an siRNA to a conserved region of a viral genome, wherein the sequence is operably linked to a promoter. The germ cell can contain a construct that comprises sequences encoding multiple siRNAs to the viral genome. Examples of viral sequences which can be used are described *supra.* In one embodiment, the virus sequence is foot-and-mouth disease virus (FDMV). In another embodiment, the virus sequence is avian influenza virus.

The invention provides for a method of generating a germ cell of a non-human vertebrate wherein the germ cell contains a construct containing a sequence encoding an siRNA to a conserved region of a viral'genome. To practice the method of the invention, one of skill in the art would incubate the germ cell with a construct comprising a sequence encoding an siRNA to.a conserved region or a viral genome wherein the sequence is operably linked to a promoter under conditions that cause the construct to be taken into the cell. In one embodiment, the construct is integrated into the host cell genome using the REMI technology.

In one aspect, the germ cell contains multiple constructs comprising a sequence encoding multiple siRNAs to conserved regions of the viral genome, wherein the sequence is operably linked to a promoter. In another embodiment, the germ cell of a non-human vertebrate is a non-human mammal. Non-limiting examples of non-human vertebrates include chicken, duck, geese, fowl, cattle, cows, pigs, fish, sheep, goats, and horses. In another embodiment, the vertebrate is of an avian species. In another aspect, the germ cell contains one vector containing several genes under the control of different promoters.

In one embodiment, the germ cell contains a construct comprising a sequence encoding an siRNA which targets Foot and Mouth Disease Virus (FMDV). Examples of such target sequences are: i). 5'-CCTGTCGCTTTGAAAGTGAAAGC-3' (SEQ ID NO:1) at nt 4900-4922, located in the 3B region; (ii) 5'-GAGATTCCAAGCTACAGATCACTTTACCTGCGTTGGGTGAACGCCGTGTGCGGT GACGC-3' (SEQ ID NO:2) at nt 6934-6992, located in the 3D region; and (iii) 5'-GACGAGTACCGGCGTCTCTTTGAGCC-3' (SEQ ID NO:3) at nt 6892-6917, located in the 3D region. All positions refer to the FMDV serotype O1 (G) sequence (GenBank accession no. AF189157).

In one aspect, the germ cell contains a construct comprising a sequence encoding an siRNA which targets avian influenza. In embodiments of this aspect, the germ cell contains a construct wherein the conserved sequence is selected from the any of the sequences depicted in Figures 1-16.

In another aspect, the germ cell contains a construct comprising a sequence encoding an siRNA which targets Marek's disease virus (MDV). In any of the above, the germ cell is sperm.

### Construction of Transgenic Animals

Constructs as described above are then introduced into animal cells by using any number of standard techniques, e.g., lipofection. However, some of the older techniques, such as lipofectin, yield a very low success rate. The inventors have found that a superior way of generating successful introduction of these siRNA-encoding constructs is to use restriction enzyme mediated integration ("REMI"), as taught in WO 99/42569. Accordingly, in one embodiment, a polynucleotide encoding siRNA is introduced into animal's germ cells (e.g., sperm) using REMI. Animals resulting from the use of these germ cells are rendered resistant to diseases. These transgenic animals can be bred to create an entire stock of virus resistant animals. In case of poultry, the viruses that the siRNA targets include, but are not limited to, Marek's disease, gumboro, and avian influenza.

The number of siRNA introduced into an animal's cell (*e.g*., germ cell) can vary. In one embodiment, one siRNA construct is introduced. In other embodiment, 1 or more siRNA is introduced. A skilled artisan will appreciate that different combinations of siRNA can be introduced based on the viral disease being targeted.

The invention provides for the generation of a non-human vertebrate that is resistant to a viral disease, wherein the majority of cells in the vertebrate comprise a sequence encoding an siRNA to a conserved region of a genome of a virus causative of the disease, wherein the sequence is operably linked to a promoter. Examples of viral sequences are discussed *supra.* In one embodiment, the construct comprises sequences encoding multiple siRNAs to conserved regions of the viral genome. In another embodiment, the cells contain multiple constructs comprising a sequence encoding multiple siRNAs to conserved regions of the viral genome. In another embodiment, the non-human vertebrate is a non-human mammal.

In one embodiment, the majority of the cells in the non-human vertebrate contains a construct comprising a sequence encoding an siRNA which targets Foot and Mouth Disease Virus (FMDV). Examples of such target sequences are: i). 5'-CCTGTCGCTTTGAAAGTGAAAGC-3' (SEQ ID NO: 1) at nt 4900-4922, located in the 3B region; (ii) 5'-GAGATTCCAAGCTACAGATCACTTTACCTGCGTTGGGTGAACGCCGTGTGCGGT GACGC-3' (SEQ ID NO:2) at nt 6934-6992, located in the 3D region; and (iii) 5'-GACGAGTACCGGCGTCTCTTTGAGCC-3' (SEQ ID NO:3) at nt 6892-6917, located in the 3D region. All positions refer to the FMDV serotype O1 (G) sequence (GenBank accession no. AF189157).

Non-limiting examples of non-human vertebrates include chicken, duck, geese, fowl, cattle, cows, pigs, fish, sheep, goats, and horses. In another embodiment, the vertebrate is of an avian species. In yet another embodiment, the non-human vertebrate has cells containing a construct encoding siRNA wherein the conserved sequence is selected from the any of the sequences depicted in Figure 1-16. In another embodiment, the non-human vertebrate is resistant to MDV.

The invention provides method for generating a non-human vertebrate that is resistant to viral disease comprising the steps of: (a) incubating the germ cell described *supra* under conditions that it forms a diploid cell; and (b) incubating the diploid cell of (a) under conditions that it forms a non-human vertebrate.

The invention also contemplates generation of a non-human vertebrate resistant to viral diseases by means of cloning. In such cases, one or more siRNA would be inserted into the genome of the cell to be cloned. Various methods of this technology are commercially available, for example, Advanced Cell Technology's nuclear transplantation protocols.

### Applications

The invention provides herein a method for creating poultry that are resistant to viral diseases. These diseases include, but are not limited to, Newcastle disease, West Nile fever, fowlpox, avian infectious bronchitis, avian encephalomyelitis, avian leukosis, duck virus hepatitis, and duck virus enteritis.

The invention provides herein a method for creating cattle that are resistant to viral diseases. These diseases include, but are not limited to: lumpy skin disease, infectious bovine rhinotracheitis, bovine virus diarrhoea, bovine leukosis, Rift Valley fever, Rinderpest, and bluetongue.

The invention provides herein a method for creating sheep and goat that are resistant to viral diseases. These diseases include, but are not limited to: Peste des petits ruminants (PPR), sheep pox and goat pox, contagious pustular dermatitis (ecthyma), border disease, bluetongue, Maedi-visna, and Rift Valley fever.

The invention provides herein a method for creating horses that are resistant to viral diseases. These diseases include, but are not limited to: equine influenza, African horse sickness, and Venezuelan equine encephalomyelitis.

The invention provides herein a method for creating fish that are resistant to viral diseases. These diseases include, but are not limited to spring viremia of carp

The invention can be applied to pigs to create resistance against classical swine fever disease, African swine fever disease, transmissible gastroenteritis (TGE) and foot-and-mouth disease. In general, the teachings herein can be generally applied to any animal that is susceptible to viral disease.

In the case of the large poultry industry that stands to sustain large economic losses due to illness, the practical solution lies in the implementation of the siRNA technology to create broiler and layer breeders resistant against avian influenza and Marek's disease, infectious bursal disease and other avian viruses. The use of the invention disclosed herein provides for a safe source of poultry for consumers and the general population as the spread of avian viruses from birds to human is halted.

The following are provided to illustrate, but not to limit, the invention.

### EXAMPLES

### Example 1 Creating Animals Resistant to Foot and Mouth Disease

Transgenic animals susceptible to foot-and-mouth disease are generated by inserting siRNA constructed from viral sequences of foot-and-mouth disease virus (FMDV) into germ cells of the animals. Generally, animals which are susceptible to FMDV are cloven-hoof animals.

### Construction of foot-and-mouth siRNA

To construct the siRNA, the target viral sequence is first identified. One way this can be accomplished is by performing local homology analysis on FMDV sequences from different strain or serotypes and identifying short stretches of sequence homology. In one embodiment, the homology is 100%, i.e., exactly the same sequence across all serotypes. In other embodiment, a difference in 1 or 2 base pairs is seen. Homology analysis is accomplished by using publicly available or commercially available programs (e.g., PILEUP and PRETTY programs in the GCG package. Homology searches are optionally conducted with publicly available sequences (e.g., Genbank) using the PILEUP and PRETTY programs or FASTA program (Pearson et al., PNAS 85: 2444-2448 (1988)).

Once short stretches of homology are identified, siRNA are synthesized using commercially available services. siRNA are synthesized in varying lengths. Some siRNA are 19 bp in length, while others are 20, 21, 22, 23, 24, 25, 26, or 27 bp in length. Each of these siRNA are either 100% conserved across the serotypes or have 1-2 bp difference in each siRNA. These conserved sequences are operably linked to at least one promoter in a vector suitable for delivery to a germ cell. Another alternative is to use siRNA that have been already synthesized, see, for example, the siRNA disclosed in Kahana et al. J. Gen. Virol. 85: 3213-3217 (2004).

These siRNA constructs are then inserted into germ cells (e.g., sperm or ova) of cloven-hoof animals using the REMI technique disclosed in WO 99/42569 to produce a germ cell with stably integrated siRNA. The germ cells are then used to produce transgenic animals by methods known in the art (*e.g.,* in vitro fertilization, artificial insemination) to produce an animal that is resistant to foot-and-mouth disease.

### Example 2 Generation of Sperm Containing siRNA for Akabane virus

A sperm line from a non-human vertebrate is generated by incubating the sperm with a construct comprising a sequence encoding an siRNA to a conserved region of Akabane virus wherein the sequence is operably linked to a promoter under conditions that cause the construct to be taken into the cell. Non-limiting examples include using lipofectin or a similar mechanism, calcium chloride, or protamine. In an alternative, the construct is stably integrated into the host cell genome using REMI technology. The siRNAs are designed according to two criteria: regions sharing high homology among the different isolates, and high likeness of silencing activity as determined by siRNA target-finding programs. The use of siRNA molecules targeted toward conserved sequences should ensure the ability of these molecules to cleave most if not all viruses of the same strain, regardless of their origin. The second and more important reason is that the conservation of a sequence indicates a strong selective pressure against change. The selective pressure would be expected to keep these target regions unchanged, whereas if they changed they might suppress the antiviral activity of the siRNAs molecules.

### Example 3 Generation of Sperm Containing siRNA for Foot-to-Mouth Disease virus

A sperm line from a non-human vertebrate is generated by incubating the sperm with a construct comprising a sequence encoding an siRNA to a conserved region of Foot-to-Mouth Disease virus wherein the sequence is operably linked to a promoter under conditions that cause the construct to be taken into the cell. Non-limiting examples include using lipofectin or a similar mechanism, calcium chloride, or protamine. In an alternative, the construct is stably integrated into the host cell genome using REMI technology.

### Example 4 Generation of Sperm Containing siRNA for Avian Influenza virus

A sperm line from a non-human vertebrate is generated by incubating the sperm with a construct comprising a sequence encoding an siRNA to a conserved region of avian influenza virus wherein the sequence is operably linked to a promoter under conditions that cause the construct to be taken into the cell. Non-limiting examples include using lipofectin or a similar mechanism, calcium chloride, or protamine. In an alternative, the construct is stably integrated into the host cell genome using REMI technology.
Also provided herein are the following aspects:
1. A germ cell of a non-human vertebrate containing a construct comprising a sequence encoding an siRNA to a conserved region of a viral genome, wherein the sequence is operably linked to a promoter.
2. The germ cell of aspect 1 wherein the construct comprises sequences encoding multiple siRNAs to the viral genome.
3. The germ cell of aspect 1 wherein the cell contains multiple constructs comprising a sequence encoding multiple siRNAs to conserved regions of the viral genome, wherein the sequence is operably linked to a promoter.
4. The germ cell of aspect 1, or aspect 2, or aspect 3 wherein the vertebrate is a non-human mammal.
5. The germ cell of aspect 4 wherein the virus is Foot and Mouth Disease Virus (FMDV).
6. The germ cell of aspect 5 wherein the conserved sequence is selected from the group consisting of SEQ ID NO: 1, SEQ ID NO: 2, and SEQ ID NO: 3.
7. The germ cell of aspect 1, or aspect 2, or aspect 3 wherein the vertebrate is of an avian species.
8. The germ cell of aspect 7 wherein the virus is avian influenza virus.
9. The germ cell of aspect 8 wherein the conserved sequence is selected from the group consisting of the sequences in Figures 1-16.
10. The germ cell of aspect 7 wherein the virus is Marek's disease virus (MDV).
11. The germ cell of aspect 1 or aspect 2, or aspect 3, or aspect 4, or aspect 5, or aspect 6, or aspect 7, or aspect 8, or aspect 9, or aspect 10, wherein the germ cell is sperm.
12. A non-human vertebrate that is resistant to a viral disease, wherein the majority of cells in the vertebrate comprise a sequence encoding an siRNA to a conserved region of a genome of a virus causative of the disease, wherein the sequence is operably linked to a promoter.
13. The non-human vertebrate of aspect 12, wherein the construct comprises sequences encoding multiple siRNAs to conserved regions of the viral genome.
14. The non-human vertebrate of aspect 12, wherein the cell contains multiple constructs comprising a sequence encoding multiple siRNAs to conserved regions of the viral genome.
15. The non-human vertebrate of aspect 12, or aspect 13, or aspect 14, wherein the vertebrate is a non-human mammal.
16. The non-human vertebrate of aspect 15, wherein the viral disease is FMDV.
17. The non-human vertebrate of aspect 16, wherein the conserved sequence is selected from the group consisting of SEQ ID NO: 1, SEQ ID NO: 2, and SEQ ID NO: 3.
18. The non-human vertebrate of aspect 12, or aspect 13, or aspect 14, wherein the vertebrate is of an avian species.
19. The non-human vertebrate of aspect 18 wherein the virus is avian influenza virus.
20. The non-human vertebrate of aspect 19 wherein the conserved sequence is selected from the group consisting of the sequences in Figures 1-16.
21. The non-human vertebrate of aspect 12 wherein the virus is MDV.
22. A method of generating a germ cell of a non-human vertebrate wherein the germ cell is that according to aspect 1, comprising incubating the germ cell with a construct comprising a sequence encoding an siRNA to a conserved region or a viral genome wherein the sequence is operably linked to a promoter under conditions that cause the construct to be taken into the cell.
23. The method of aspect 22 wherein the construct is integrated into the host cell genome.
24. A method for generating a non-human vertebrate according to aspect 12
   (a) incubating the germ cell of aspect 1, or aspect 2, or aspect 3, or aspect 4, or aspect 5, or aspect 6, or aspect 7, or aspect 8, or aspect 9, or aspect 10, or aspect 11 under conditions that it forms a diploid cell; and
   (b) incubating the diploid cell of (a) under conditions that it forms a non-human vertebrate.

## Claims

1. A transgenic non-human organism that is resistant to a viral disease, wherein the majority of cells in the organism comprise a sequence encoding an siRNA to a conserved region of a genome of a virus causative of the disease, wherein the sequence is operably linked to a promoter.

2. A transgenic organism according to claim 1 wherein the organism is an avian selected from the group consisting of chicken, duck, and geese.

3. The avian of claim 2 wherein the viral disease is any one or more of: New Castle disease, West Nile fever, fowlpox, avian infectious bronchitis, avian encephalomyelitis, avian leukosis, duck virus hepatitis, and duck virus enteritis.

4. A transgenic organism according to claim 1 wherein the organism is a pig.

5. The pig of claim 4 wherein the viral disease is selected from the group consisting of classical swine fever disease, African swine fever disease, transmissible gastroenteritis (TGE) and foot-and-mouth disease.

6. A transgenic organism according to claim 1 wherein the organism is a cattle.

7. The cattle of claim 6 wherein the viral disease is selected from the group consisting of lumpy skin disease, infectious bovine rhinotracheitis, bovine virus diarrhoea, bovine leukosis, Rift Valley fever, Rinderpest, bluetongue, and foot and mouth disease.

8. A transgenic organism according to claim 1 wherein the organism isa sheep or goat.

9. The sheep or goat of claim 8 wherein the viral disease is selected from the group consisting of Peste des petits ruminants (PPR), sheep pox and goat pox, contagious pustular dermatitis (ecthyma), border disease, bluetongue, Maedi-visna, and Rift Valley fever.

10. A transgenic organism according to claim 1 wherein the organism is a horse.

11. The horse of claim 10 wherein the viral disease is selected from the group consisting of equine influenza, African horse sickness, and Venezuelan equine encephalomyelitis.

12. A transgenic organism according to claim 1 wherein the organism is a fish.

13. The fish of claim 12 wherein the viral disease is spring viremia of carp.

14. A stock of virus resistant non-human vertebrates comprising a plurality of the non-human vertebrates of any one of claims 1-13.
